# EUROPEAN PATENT APPLICATION

(11) **EP 3 670 666 A1**
(43) Date of publication of application: **24.06.2020**
(21) Application number: 18214456.8
(22) Date of filing: 20.12.2018
(51) Int. Cl.: C12Q 1/6804, C12Q 1/6832, C12Q 1/6841

(54) **METHOD FOR OPTICAL IMAGING A TARGET MOLECULE IN A SAMPLE, A PROTEIN-OLIGONULEOTIDE CONJUGATE FOR USE IN THE METHOD AND A KIT FOR CARRYING OUT THE METHOD**

(71) Applicant: Freie Universität Berlin, 14195 Berlin (DE)
(72) Inventor: EWERS, Helge, 12209 Berlin (DE); FÜRSTE, Jens-Peter, 14165 Berlin (DE); GEERTSEMA, Hylkje, 12557 Berlin (DE)
(74) Representative: Gulde & Partner

(57) **Abstract**

The invention concerns a method for optical imaging by delivery of a dye via a base-pairing mechanism, wherein a first strand being delivered to a target structure, and a complimentary second strand carrying a dye molecule. According to the invention the two pairing strands comprises non-naturally occurring oligonucleotide.

## Description

Far-field fluorescence microscopy has seen major advances since the advent of methods that circumvent the classical diffraction limit, e.g., super-resolution microscopy. Several methods of implementation rely on the switching molecules between fluorescent ON- and OFF-states to allow consecutive localization of individual molecules to assemble an image from the resulting accurate localizations of single molecules. Switching is traditionally obtained in one of two ways: "targeted" switching actively confines the fluorescence excitation to an area smaller than the diffraction of light (e.g., stimulated emission depletion microscopy, or STED), whereas "stochastic" switching uses photoswitchable proteins (photoactivated localization microscopy, or PALM), photoswitchable organic dyes (stochastic optical reconstruction microscopy, or STORM) or molecules that become detectable by association or binding (PAINT, IRIS, DNA-PAINT). Although these methods offer enhanced spatial resolution, they tend to require either expensive instrumentation or highly specialized experimental conditions, and thus have not yet been developed into common biological laboratory techniques.

WO 2015/ 017586 A1 provides, inter alia, methods, compositions (e.g., conjugates) and kits for imaging, at high or low spatial resolution, targets (e.g., biomolecules) of interest in, for example, a cellular environment. The methods, compositions and kits of the present disclosure take advantage of repetitive, transient binding of short, labeled (e.g., fluorescently labeled) oligonucleotides (e.g., DNA oligonucleotides), or "imager" strands, to complementary "docking" strands which are attached to targets of interest, in some embodiments, through an intermediate molecule such as an antibody such as a primary or a secondary antibody, to obtain stochastic switching between fluorescent ON- and OFF-states. In the unbound state, only background fluorescence from partially quenched imager strands is observed. This is considered an "OFF" state. Upon binding and immobilization of an imager strand, fluorescence emission is detected using, for example, total internal reflection (TIR) or highly inclined and laminated optical sheet (HILO) microscopy. This is considered an "ON" state. In general, the methods, compositions and kits as provided herein increase the imaging resolution and thus the sensitivity of detection. In some aspects, they also increase the specificity as well as the number of utilizable fluorophores available for detecting targets of interest including but not limited to, e.g., naturally-occurring biomolecules.

By linking a short docking strand to a binding partner (e.g., a protein-binding moiety or a nucleic acid-binding moiety, whether primary or secondary), such as an antibody including a primary and a secondary antibody, different species of targets (e.g., biomolecules, optionally in a cellular environment) can be labeled and subsequently detected by introducing fluorescently-labeled imager strands that are complementary to and bind to the docking strands through transient Watson-Crick interactions. Unlike existing detection methods, the methods of the present invention are not limited by the number of spectrally distinct fluorophores available for detecting distinct targets (e.g., biomolecules). Rather, the programmability of nucleic acid (e.g., DNA and/or RNA) molecules and sequential time-lapsed imaging are used herein to provide images of up to hundreds of distinct species of targets using, in some embodiments, only a single optimized fluorophore. Further, these different species of targets (e.g., biomolecules) can be quantified using predictable kinetics of binding of single fluorescently-labeled imager strands to their complementary target docking strands.

Disadvantageously, the method shows a significant amount of background fluorescence resulting from imager strands bound to naturally occurring (genomic and other) nucleotides exhibiting nucleotide-sequences that are complementary to the imager strand.

This can be reduced by increasing the amount of bases in the imager strand, so that the likelihood of accidental match with a section of DNA in the sample decreases.

However, the increase in the number of bases is also associated with an increase in the binding period, which significantly prolongs the measurement time and may result in overlap of detected molecules. In addition, the background noise is only reduced and not avoided.

The problem to be solved is now to suppress the background noise as much as possible while maintaining the optimal number of bases per adapter and/or imager strand to maintain effective measurement time.

This problem will be solved by a method for optical imaging a target molecule in a sample, a protein-nucleotide used in the method and a kit according to the independent claims.

Accordingly, a first aspect of the present invention is a method for optical imaging by delivery of a dye via a base-pairing mechanism, wherein a first strand being delivered to a target structure, and a complimentary second strand carrying a dye molecule characterized in that the two pairing strands comprise non-naturally occurring oligonucleotide.

As a result, imager strands used in imaging methods to examine samples of natural origin have no binding partners in the sample other than the adapter strands added as planned. This means that the imager strands are linked exclusively to the adapter strands provided for this purpose and built into the target molecules in a planned manner. A random linking with natural bases of the sample does not occur, since the sample contains only natural nucleic acid in addition to the introduced adapter strands, which are designed to being compatible with the imager strands.

Consequently, the imager strands only bind to the target molecules and there is no noise.

Advantageously, the background noise is not only suppressed, but rather its source is removed. As a result, image quality and yield are improved. Further, the amount of equipment and image post-processing can be reduced. The method is also more tolerant and enables the detection of smaller concentrations of target molecules in the sample.

In an embodiment of the invention it is preferred that the non-naturally occurring oligonucleotide is a left handed oligonucleotide, especially a left handed DNA. Left-handed DNA has identical physicochemical properties as right-handed DNA, with the exception of the direction of rotation of the double helix. Due to the different direction of rotation, the binding of corresponding base pairs, which are arranged next to each other in the strand, is hindered sterically. The result is an absence of binding of complementary sequences to one another of right-handed and left-handed DNA strands with a length of more than two base pairs. Left-handed DNA will thus not bind to any natural DNA present in biological material.

Alternatively or additionally, further modifications of the DNA may be planned. Thus, for example, a further version of the inventive method proposes that the non-naturally occurring oligonucleotide is built by replacing units or ions of the backbone by derivatives thereof or chemical compatible ions, especially replacing phosphor-ions by arsenic-ions. The difference of ion diameter results in no bonding between naturally occurring nucleotides and the modified ones.

In a preferred embodiment according to the invention the first strand is an adapter strand which is capable of transiently binding to the second strand. That is, the binding is only temporally depending on the binding strength between first and second strand which leads to the fact that the dye is localized on a certain position for a certain time and can be measured until the linkage between the two strands re-opened and the second strand linked to another first strand bonded to an other target position. Again the dye is detectable on that position for a certain time.

Even with sub-stoichiometric concentration of the second strands, it can be ensured that all positions of the target molecule marked with the first strands, i.e. the adapter strands, are made visible. The duration of the binding and thus the requirement for the temporal resolution of the measurement can be set by the length of the strands, in particular the second strands.

Therefore, it is also preferred that the complementary labeled imager strand is about 4 to about 30 nucleotides, especially 7 to 20, preferably 8 to 10 in length, till this leads to highly resolvable visualization. In this and other aspects and embodiments described herein, the adapter strand may comprise a plurality of domains, each complementary to a labeled imager strand. The domains may be identical in sequence (and thus will bind to the identical imager strands) or they may be of different sequence (and thus may bind to imager strands that are not identically labeled). Such domains may also be referred to herein as binding sites for imager strands.

In some embodiments, a docking strand includes at least two or at least three domains, each respectively complementary to a labeled imager strand.

Further it is preferred, that the second strand is an imager strand carrying a dye, wherein the label comprises one of a fluorophene, a phosphorescene, a quantum dot, a nanoparticle or a nanodiamond.

Alternatively of / additionally to each of the aforementioned embodiments it is preferred, that the dye is imaging active if the second strand is linked to a corresponding first strand, and optionally imaging inactive if the second strand is not linked to a corresponding first strand. Advantageously, this further reduces background noise. In this embodiments background noise which is caused by unlinked imager strands is reduced. This background noise is generally lower than that of the randomly falsely bound imager strands, since unbound imager strands move quickly and cannot be localized with measurement methods of usual time resolution. Rather, this background noise creates an increased baseline by measuring received signals.

The reduced background noise enables to detect more than one sort of imager strands coupled to differentiating dyes. Accordingly, it is preferred that a further pair of imager and adapter strands are present in the method which differs from the first pair in the target structure and the corresponding nucleotide sequence. They may also differ in the color of the dye for simultaneous imaging of spectrally resolvable dyes or may not differ in the dye for sequential imaging in the absence of chromatic aberration, which will allow for highly multiplexed labeling.

In further preferred embodiments the target is linked to the first strand through an intermediate linker, wherein the intermediate linker especially comprises biotin and/or streptavidin.

In more preferred embodiments the adapter strand is bound to a target-recognizing antibody, antigen-binding antibody fragment, nanobody, or a peptide aptamer. In some embodiments, an antibody is a monoclonal antibody. This leads to a highly specific marking of the target molecule and thus to a high quality and high yield of imaging.

It shows that it is advantageous if the target-recognizing antibody or nanobody provides a biotin binding site, a streptavidin binding to biotin-bound antibody or nanobody, whereby the adapter strand with a biotin binding site is capable to strongly bind to the streptavidin.

Another aspect of the present invention is a protein-oligonucleotide conjugate which is formed as an intermediate product in the inventive method according to the invention as well as a target bound to such conjugate. The conjugate comprising a target linked to an adapter strand that is capable of transiently binding to a complementary labeled imager strand, characterized in that the adapter strand and the imager strand comprise non-naturally occurring oligonucleotide.

A further aspect of the present invention concerns a Kit comprising a plurality of protein-nucleic acid conjugates, or a number of components that can be assembled into a labeling reagent from dye-coupled nucleic acids and coupling reagents, optionally wherein at least one of the protein-nucleic acid conjugates is bound to at least one target.

### SUMMARY OF THE INVENTION

The present disclosure provides, inter alia, methods, compositions (e.g., conjugates) and kits for imaging, at high or low spatial resolution, targets (e.g., biomolecules) of interest in, for example, a cellular environment. The methods, compositions and kits of the present disclosure take advantage of repetitive, transient binding of short, labeled (e.g., fluorescently labeled) oligonucleotides (e.g., DNA oligonucleotides), or "imager" strands, to complementary "docking" strands, which are attached to targets of interest, in some embodiments, through an intermediate molecule such as an antibody such as a primary or a secondary antibody, to obtain stochastic switching between fluorescent ON- and OFF-states. In the unbound state, only background fluorescence from partially quenched imager strands is observed. This is considered an "OFF" state. Upon binding and immobilization of an imager strand, fluorescence emission is detected using, for example, total internal reflection (TIR) or highly inclined and laminated optical sheet (HILO) microscopy. This is considered an "ON" state. In general, the methods, compositions and kits as provided herein increase the imaging resolution and thus the sensitivity of detection. In some aspects, they also increase the specificity as well as the number of utilizable fluorophores available for detecting targets of interest including but not limited to, e.g., naturally-occurring biomolecules.

By linking a short adapter strand to a binding partner (e.g., a protein-binding moiety or a nucleic acid-binding moiety, whether primary or secondary), such as an antibody including a primary and a secondary antibody, different species of targets (e.g., biomolecules, optionally in a cellular environment) can be labeled and subsequently detected by introducing fluorescently-labeled imager strands that are complementary to and bind to the docking strands through transient Watson-Crick interactions. Unlike existing detection methods, the methods of the present disclosure are not limited by the number of spectrally distinct fluorophores available for detecting distinct targets (e.g., biomolecules). Rather, the programmability of nucleic acid (e.g., DNA and/or RNA) molecules and sequential time-lapsed imaging are used herein to provide images of up to hundreds of distinct species of targets using, in some embodiments, only a single optimized fluorophore. Further, these different species of targets (e.g., biomolecules) can be quantified using predictable kinetics of binding of single fluorescently-labeled imager strands to their complementary target docking strands.

According to the invention the strands of the imager strand and the adapter strands, respectively, are non-naturally occurring oligonucleotides.

In some instances, the methods can be used to generate super-resolution images, significant even without the need for a super-resolution microscope. It should be understood that while the methods, compositions and kits as provided herein may be described for use in super-resolution imaging, they may also be used, in some embodiments, for imaging that does not require super-resolution. Thus, in some embodiments, the methods, compositions and kits of the present disclosure may be used for imaging in general.

In some aspects, provided herein is a protein-nucleic acid conjugate, comprising a protein linked to a docking strand that is capable of transiently binding to a complementary labeled imager strand. In some aspects, provided herein is a protein-nucleic acid conjugate, comprising a protein linked to a docking strand that is transiently bound to a complementary labeled imager strand. Imager strands, in some embodiments, are labeled with a detectable label. The detectable label may be, for example, a fluorescent label or other detectable label, e.g., gold nanoparticles. While various aspects and embodiments herein refer to fluorescently-labeled imager strands, it should be understood that such fluorescent labels, in many instances, can be interchanged with other detectable labels. Thus, in some embodiments, a fluorescently-labeled imager strand (which may be detected by, for example, fluorescent microscopy) may be interchanged with an imager strand labeled with, for example, gold nanoparticles (which may be detected by, for example, dark field microscopy). It is also to be understood that the docking strands may be capable of transiently binding a plurality of complementary labeled strands (e.g., the docking strand may comprise a plurality of binding sites for complementary labeled strands).

In some embodiments, a method may be carried out involving a plurality of docking strand and imager strand pairs. Such a method can be used to detect a plurality of targets (e.g., with each docking strand-imager strand pair corresponding to one target). The docking strand-imager strand pairs in the plurality must share an approximately equal probability of hybridizing under a single environment or condition (as defined, for example, by temperature, salt concentration, strand molarity, etc.), such that if there is an observed difference between the level of binding (and thus the detection) of a population of imager strands, an end user can conclude that such difference is a function of the amount of docking strand and thus ultimately the amount of target.

In some embodiments, the docking and imager strands are typically selected such that their bound states have a thermal stability in the range of about +/- 0.5 kcal/mol. With this range of thermal stability, it is possible to select at least 200 orthogonal (e.g., different) sequences to be used in these multiplexing methods.

In some embodiments, a protein is an antibody such as a primary antibody or a secondary antibody, an antigen-binding antibody fragment, or a peptide aptamer. In some embodiments, a protein is linked to the docking strand through an intermediate linker.

In some embodiments, the intermediate linker comprises biotin and streptavidin.

In some embodiments, the target is a protein.

In some embodiments, the target is a nucleic acid (e.g., DNA or RNA).

In some aspects, provided herein is a plurality of protein-nucleic acid conjugates.

In some embodiments, the plurality comprises at least two subsets of the protein-nucleic acid conjugates, and the protein-nucleic acid conjugates of each subset bind to different targets.

In some aspects, provided herein is a composition or kit comprising a plurality of protein-nucleic acid conjugates, optionally wherein at least one of the protein-nucleic acid conjugates is bound to at least one target.

In some aspects, provided herein is a composition or kit comprising at least one protein-nucleic acid conjugate that comprises a protein linked to a docking strand, optionally wherein the at least one protein-nucleic acid conjugate is bound to a target, and at least one complementary labeled, optionally fluorescently labeled, imager strand that is transiently bound to or is capable of transiently binding to the at least one protein-nucleic acid conjugate.

In some embodiments, a composition or kit comprises at least two complementary labeled, optionally fluorescently labeled, imager strands, wherein the at least two complementary labeled imager strands are identical.

In some embodiments, the composition or kit comprises at least two complementary labeled imager strands, wherein the at least two complementary labeled imager strands are different.

In some embodiments, the number of complementary labeled, optionally fluorescently labeled, imager strands is less than, greater than or equal to the number of protein-nucleic acid conjugates.

In some embodiments, a composition or kit comprises at least 2, 3, 4, 5, 6, 7, 9 or 10 different complementary labeled, optionally fluorescently labeled, imager strands.

In some embodiments, the composition or kit comprises at least 50 or at least 100 different complementary fluorescently-labeled imager strands.

In some aspects, provided herein is a composition or a kit comprising a (e.g., one or more) docking strand and an (e.g., one or more) imager strand. The docking strand may be modified to include an affinity label, thereby facilitating its subsequent attachment to one or more binding partners, such as an antibodies. For example, the docking strand may be biotinylated or it may be attached to avidin or streptavidin. Other affinity labels can be used instead. The imager strands may be labeled, such as fluorescently labeled. The imager strands may be a plurality of identical imager strands (e.g., with respect to sequence and label) or they may be a plurality of different imager strands (e.g., with respect to sequence and label). The composition or kit may further comprise a target-specific binding partner, such as an antibody. It is to be understood that the components may be bound to each other or they may be unbound, including physically separated from each other, in such compositions and kits. These and other compositions and kits may further comprise one or more buffers including oxygen scavengers.

In some aspects, provided herein is a composition or kit comprising an antibody-nucleic acid conjugate, wherein the antibody is a "secondary antibody" having specificity for an antibody, typically specificity for a particular isotype or an Fc domain of an antibody from a particular species (e.g., a mouse antibody that is specific for a human IgG1 antibody). The nucleic acid in the conjugate is a docking strand, as described herein. The composition or kit may further comprise one or more imager strands (or one or more subsets or populations of imager strands), as described herein. These and other compositions and kits may further comprise one or more buffers including oxygen scavengers or reagents preventing photobleaching or blinking.

In some aspects, the present disclosure provides an antibody-DNA conjugate, comprising a monoclonal antibody linked to a docking strand that is bound to a complementary labeled, optionally fluorescently labeled, imager strand, wherein the antibody and the docking strand are each biotinylated and linked to each other through an avidin or streptavidin linker or a biotin-streptavidin linker.

In some aspects, provided herein is an aptamer-nucleic acid conjugate, comprising a nucleic acid aptamer linked to a docking strand that is transiently bound to a complementary labeled, optionally fluorescently labeled, imager strand.

In some aspects, provided herein is a method of detecting a target in a sample, the method comprising contacting a sample with (a) at least one protein-nucleic acid conjugate that comprises a protein linked to a docking strand and (b) at least one fluorescently-labeled imager strand that is complementary to and transiently binds to the docking strand of the at least one protein-nucleic acid conjugate, and determining whether the at least one protein-nucleic acid conjugate binds to the target in the sample.

In some embodiments, the determining step comprises imaging transient binding of the at least one fluorescently-labeled imager strand to the docking strand of the at least one protein-nucleic acid conjugate.

In some aspects, provided herein is a method of detecting a target in a sample, the method comprising contacting a sample with (a) at least one protein-nucleic acid conjugate that comprises a protein linked to a docking strand and (b) at least one fluorescently-labeled imager strand that is complementary to and transiently binds to the docking strand of the at least one protein-nucleic acid conjugate, and imaging transient binding, optionally using time-lapsed imaging, of the at least one fluorescently-labeled imager strand to the docking strand of the at least one protein-nucleic acid conjugate.

In some embodiments, a sample is a cell or cell lysate, and/or the target is obtained from a cell or cell lysate.

In some aspects, provided herein is a method of detecting at least one or at least two targets in a sample, the method comprising contacting a sample with (a) at least two protein-nucleic acid conjugates, each comprising a protein linked to a docking strand, and (b) at least two labeled (optionally spectrally distinct, or fluorescently labeled, or spectrally distinct and fluorescently labeled) imager strands that are complementary to and transiently bind to respective docking strands of the at least one, or at least two, different protein-nucleic acid conjugates and determining whether the at least two protein-nucleic acid conjugates bind to at least two targets in the sample. In some embodiments, the determining step comprises, in the following order, imaging transient binding of one of the at least two labeled imager strands to a docking strand of one of the at least two protein-nucleic acid conjugates to produce a first image (e.g., of a fluorescent signal), and imaging transient binding of another of the at least two labeled imager strands to a docking strand of another of the at least two protein-nucleic acid conjugates to produce at least one other image (e.g., of a fluorescent signal). In some embodiments, the method further comprises combining the first image and the at least one other image to produce a composite image of signal (e.g., fluorescent signal), wherein the signal of the composite image is representative of the at least two targets.

In some aspects, provided herein is a method of detecting at least one, or at least two, protein targets in a sample, comprising (a) contacting a sample with at least two protein-nucleic acid conjugates, each comprising a protein linked to a docking strand and (b) sequentially contacting the sample with at least two labeled (e.g., optionally spectrally indistinct, or fluorescently labeled, or spectrally distinct and fluorescently labeled) imager strands that are complementary to and transiently bind to respective docking strands of the at least two protein-nucleic acid conjugates, and determining whether the at least one, or at least two, protein-nucleic acid conjugates bind to at least two targets in the sample. In some embodiments, a method comprises, in the following ordered steps, contacting the sample with a first protein-nucleic acid conjugate and at least one other protein-nucleic acid conjugate, contacting the sample with a first labeled, optionally fluorescently labeled, imager strand that is complementary to and transiently binds to the docking strand of the first protein-nucleic acid conjugate, imaging the sample to obtain a first image, optionally using time-lapsed imaging, removing the first labeled imager strand, contacting the sample with at least one other labeled imager strand that is complementary to and transiently binds to the docking strand of the at least one other protein-nucleic acid conjugate, and imaging the sample to obtain at least one other image, optionally using time-lapsed imaging.

In some embodiments, a method comprises, in the following ordered steps, contacting a sample with a first protein-nucleic acid conjugate, contacting the sample with a first labeled, optionally fluorescently labeled, imager strand that is complementary to and transiently binds to the docking strand of the first protein-nucleic acid conjugate, imaging the sample to obtain a first image, optionally using time-lapsed imaging, removing the first labeled imager strand, contacting the sample with at least one other protein-nucleic acid conjugate, contacting the sample with at least one other labeled, optionally fluorescently labeled, imager strand that is complementary to and transiently binds to the docking strand of the at least one other protein-nucleic acid conjugate, and imaging the sample to obtain at least one other image, optionally using time-lapsed imaging.

In some embodiments, a method further comprises determining whether the first protein-DNA conjugate binds to a first target and/or whether the at least one other protein-DNA conjugate binds to at least one other target.

In some embodiments, a method further comprises assigning a pseudo-color to the signal (e.g., fluorescent signal) in a first image, and assigning at least one other pseudo-color to the fluorescent signal in the at least one other image.

In some embodiments, a method further comprises combining a first image and the at least one other image to produce a composite image of the pseudo-colored signals, wherein the pseudo-colored signals of the composite image are representative of the at least two targets.

In some embodiments, a method further comprises determining whether the first protein DNA conjugate binds to a first target, optionally a naturally-occurring biomolecule, and/or whether the at least one other protein-DNA conjugate binds to at least one other target, optionally a naturally-occurring biomolecule.

In some embodiments, a method further comprises assigning a pseudo-color to the signal (e.g., fluorescent signal) in a first image, and assigning at least one other pseudo-color to the signal (e.g., fluorescent signal) in at least one other image.

In some embodiments, a method further comprises combining a first image and at least one other image to produce a composite image of pseudo-colored signals, wherein the pseudo-colored signals of the composite image are representative of at least one, or at least two, targets (e.g., naturally-occurring biomolecules).

In some aspects, provided herein is a method of determining the number of targets in a test sample, comprising obtaining a sample that comprises targets transiently bound directly or indirectly to labeled, optionally fluorescently labeled, imager strands, obtaining a time-lapsed image, optionally a time-lapsed diffraction-limited fluorescence image, of the sample, performing spot detection (e.g., fluorescence spot detection) and localization (e.g., through the use of Gaussian fitting) on the diffraction-limited image to obtain a high-resolution image of the sample, calibrating kₒₙ • c_{imager}, optionally using a control sample with a known number of targets, wherein kₒₙ is a second order association constant, and c_{imager} is concentration of labeled (e.g., fluorescently labeled) imager strands in the test sample, determining variable Td, optionally by fitting the fluorescence OFF-time distribution to a cumulative distribution function, and determining the number of test targets in the sample based on the equation, number of test targets = (kₒₙ • C_{imager} • τ_{d})⁻¹.

In some aspects, provided herein is a method of determining a relative amount of targets in a test sample, comprising obtaining a sample that comprises targets transiently bound directly or indirectly to labeled imager strands, obtaining a time-lapsed image of the sample, performing spot detection and localization on the image to obtain a high-resolution image of the sample, determining variable ID, and determining the relative amount of two or more test targets in the sample based on τ_{d}.

In some embodiments, test targets are protein targets.

The present disclosure provides, inter alia, methods, compositions and kits for multiplexed imaging, for example, in a cellular environment using nucleic acid-based imaging probes (e.g., DNA-based imaging probes). The methods, compositions and kits for multiplexed fluorescence imaging are not limited by the degree of resolution attained. Thus, the methods, compositions and kits as provided herein may be used for imaging in general.

In some aspects, the present disclosure further provides, inter alia, methods, compositions and kits for multiplexed super-resolution fluorescence imaging, for example, in a cellular environment using nucleic acid-based imaging probes (e.g., DNA-based imaging probes). As used herein, "super-resolution" imaging refers to the process of combining a set of low resolution images of the same area to obtain a single image of higher resolution. Many aspects of the present disclosure may be used to "switch" targets (e.g., biomolecules of interest between fluorescent ON- and OFF-states to permit consecutive, or in some instances simultaneous, localization of individual targets. A fluorescent "ON" state is a state in which fluorescence is emitted. A fluorescent "OFF" state is a state in which fluorescence is not emitted. Switching between the two states is achieved, in some embodiments, with diffusing molecules that are labeled with a detectable label (e.g., fluorescent molecules) that interact transiently with the targets using an intermediate moiety that comprises the detectable label (e.g., fluorescent molecule(s)) and binds to the target. The methods, compositions and kits of the present disclosure are useful, in some aspects, for detecting, identifying and quantifying targets of interest.

Binding partner-nucleic acid conjugates (BP-NA conjugates") of the present disclosure transiently bind to complementary labeled, optionally fluorescently labeled, imager strands. As used herein, "binding partner-nucleic acid conjugate," or "BP-NA conjugate," refers to a molecule linked (e.g., through an N-Hydroxysuccinimide (NHS) linker, maleimide-linker, Sortase reaction or similar) to a single-stranded nucleic acid (e.g., DNA) docking strand. The binding partner of the conjugate may be any moiety (e.g., antibody, affimer or aptamer) that has an affinity for (e.g., binds to) a target, such as a biomolecule (e.g., protein or nucleic acid) of interest.

In some embodiments, the binding partner is a protein. BP-NA-conjugates that comprise a protein or peptide linked to a docking strand may be referred to herein as "protein-nucleic acid conjugates" or "protein-NA conjugates". Examples of proteins for use in the conjugates of the present disclosure include, without limitation, antibodies (e.g., monoclonal monobodies), antigen-binding antibody fragments (e.g., Fab fragments), receptors, peptides and peptide aptamers. Other binding partners may be used in accordance with the present disclosure. For example, binding partners that bind to targets through electrostatic (e.g., electrostatic particles), hydrophobic or magnetic (e.g., magnetic particles) interactions are contemplated herein.

As used herein, "antibody" includes full-length antibodies and any antigen binding fragment (e.g., "antigen-binding portion-) or single chain thereof. The term "antibody" includes, without limitation, a glycoprotein comprising at least two heavy (H) chains and possibly two light (L) chains inter-connected by disulfide bonds, or an antigen binding portion thereof. Antibodies may be polyclonal or monoclonal; xenogeneic, allogeneic, or syngeneic; or modified forms thereof (e.g., humanized, chimeric).

As used herein, "antigen-binding portion" of an antibody, refers to one or more fragments of an antibody that retain the ability to specifically bind to an antigen. The antigen-binding function of an antibody can be performed by fragments of a full-length antibody. Examples of binding fragments encompassed within the term "antigen-binding portion" of an antibody include (i) a Fab fragment, a monovalent fragment consisting of the VH, VL, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VH and VL domains of a single arm of an antibody, (v) a dAb fragment (Ward et al.. Nature 341;544 546, 1989), which consists of a VH domain; and (vi) an isolated complementarity determining region (CDR) or (vii) a combination of two or more isolated CDRs, which may optionally be joined by a synthetic linker. Furthermore, although the two domains of the Fv fragment, VH and VL, are coded for by separate genes, they can be joined, using recombinant methods, by a synthetic linker that enables them to be made as a single protein chain in which the VH and VL regions pair to form monovalent molecules (known as single chain Fv (scFv); see, e.g., Bird et al. Science 242:423 426, 1988; and Huston et al. Prot. Natl. Acad. Sci. USA 85:5879-5883, 1988). Such single chain antibodies are also encompassed within the term "antigen-binding portion" of an antibody. These antibody fragments are obtained using conventional techniques known to those with skill in the art. and the fragments are screened for utility in the same manner as are intact antibodies.

As used herein, "receptors" refer to cellular-derived molecules (e.g., proteins) that bind to ligands such as, for example, peptides or small molecules (e.g., low molecular weight (<900 Dalions) organic or inorganic compounds).

As used herein, "peptide aptamer" refers to a molecule with a variable peptide sequence inserted into a constant scaffold protein (see, e.g., Baines IC, et al. Drug Discov. Today 11:334-341, 2006).

In some embodiments, the molecule of the BP-NA conjugate is a nucleic acid such as, for example, a nucleic acid aptamer. As used herein, "nucleic acid aptamer" refers to small RNA or DNA molecules that can form secondary and tertiary structures capable of specifically binding proteins or other cellular targets (see, e.g., Ni X, et al. Curr Med Chem. 18(27): 4206-4214, 2011). Thus, in some embodiments, the BP-NA conjugate may be an aptamer-nucleic acid conjugate.

As used herein a "docking strand" refers to a single-stranded nucleic acid (e.g., DNA) that is about 5 nucleotides to about 50 nucleotides in length (or is 5 nucleotides to 50 nucleotides in length). In some embodiments, a docking strand is about 4 to about 60, about 6 nucleotides to about 40 nucleotides, about 7 nucleotides to about 30 nucleotides, about 8 to about 20 nucleotides, or about 9 nucleotides to about 15 nucleotides in length. In some embodiments, a docking strand is (or is about) 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, or more nucleotides in length.

A docking strand may have one domain or more than one domain (e. g., a plurality of domains), each domain complementary to a respective imager strand. As used herein, a "docking strand domain" refers to a nucleotide sequence of the docking strand that is complementary to a nucleotide sequence of an imager strand. A docking strand, for example, may contain one, two, three, or more domains, each domain complementary to an imager strand. Each complementary imager strand can contain a distinct label (e.g., a red fluorophore, a blue fluorophore, or a green fluorophore), or all complementary imager strands can contain the same label (e.g., red fluorophores). For example, for a three-domain docking strand, the strand may contain a first domain complementary to an imager strand labeled with a red fluorophore, a second domain complementary to an imager strand labeled with a blue fluorophore, and a third domain complementary to an imager strand labeled with a green fluorophore. Alternatively, each of the three docking domains may be complementary to imager strands labeled with a red fluorophore. In some embodiments, a docking strand has at least 2, at least 3, at least 4, at least 5, or more domains, each respectively complementary to an imager strand. In some embodiments, a docking strand has 1 to 5, 1 to 10, 1 to 15, 1 to 20, 1 to 25, 1 to 50, or 1 to 100 domains, each respectively complementary to an imager strand. As used herein, an "imager strand" is a single-stranded nucleic acid (e.g., DNA) that is about 4 to about 30 nucleotides, about 5 to about 18 nucleotides, about 6 to about 15 nucleotides, about 7 to about 12 nucleotides, or about 8 to 10 nucleotides in length and is fluorescently-labeled. In some embodiments, the imager strand may be (or may be about) 4, 5, 6, 7, 8, 9, 10, 11, 12, 13, 14, 15, 16, 17, 18, 19, 20, 21, 22, 23, 24,25, 26,27, 28, 29 or 30 nucleotides in length. An imager strand of the present disclosure is complementary to and transiently binds to a docking strand. Two nucleic acids or nucleic acid domains are "complementary" to one another if they base-pair, or bind, with each other to form a double-stranded nucleic acid molecule via Watson-Crick interactions. As used herein, "binding" refers to an association between at least two molecules due to, for example, electrostatic, hydrophobic, ionic and/or hydrogen-bond interactions under physiological conditions. An imager strand is considered to "transiently bind" to a docking strand if it binds to a complementary region of a docking strand and then disassociates (unbinds) from the docking strand within a short period of time, for example, at room temperature.

In some embodiments, an imager strand remains bound to a docking strand for about 0.1 to about 10, or about 0.1 to about 5 seconds. For example, an imager strand may remain bound to a docking strand for about 0.1, about 1, about 5 or about 10 seconds.

Imager strands of the present disclosure may be labeled with a detectable label (e.g., a fluorescent label, and thus are considered "fluorescently labeled"). For example, in some embodiments, an imager strand may comprise at least one (i.e., one or more) fluorophore. Examples of fluorophores for use in accordance with the present disclosure include, without limitation, xanthene derivatives (e.g., fluorescein, rhodamine, Oregon green, eosin and Texas red), cyanine derivatives (e.g., cyanine, indocarbocyanine, oxacarbocyanine, thiacarbocyanine and merocyanine), naphthalene derivatives (e.g., dansyl and prodan derivatives), coumarin derivatives, oxadiazole derivatives (e.g., pyridyloxazole, nitrobenzoxadiazole and benzoxadiazole), pyrene derivatives (e.g., cascade blue), oxazine derivatives (e.g., Nile red, Nile blue, cresyl violet and oxazine 170), acridine derivatives (e.g., proflavin, acridine orange and acridine yellow), arylmethine derivatives (e.g., auramine, crystal violet and malachite green), and tetrapyrrole derivatives (e.g., porphin, phthalocyanine and bilirubin). Other detectable labels may be used in accordance with the present disclosure, such as, for example, gold nanoparticles or other detectable particles or moieties.

As used herein, "spectrally distinct" molecules of the present disclosure (e.g., conjugates and/or imager strands) refer to molecules with labels (e.g., fluorophores) of different spectral signal or wavelength. For example, an imager strand labeled with a Cy2 fluorophore emits a signal at a wavelength of light of about 510 nm, while an imager strand labeled with a Cy5 fluorophore emits a signal at a wavelength of light of about 670 nm. Thus, the Cy2-labeled imager strand is considered herein to be spectrally distinct from the Cy5-labeled imager strand. Conversely, "spectrally indistinct" molecules of the present disclosure herein refer to molecules with labels having the same spectral signal or wavelength - that is, the emission wavelength of the labels cannot be used to distinguish between two spectrally indistinct fluorescently labeled molecules (e.g., because the wavelengths are the same or close together).

The BP-NA conjugates (e.g., protein-nucleic acid conjugates) of the present disclosure may, in some embodiments, comprise an intermediate linker that links (e.g., covalently or non-covalently) the molecule to a docking strand. The intermediate linker may comprise biotin and/or streptavidin. For example, in some embodiments, an antibody and a docking strand may each be biotinylated (i.e., linked to at least one biotin molecule) and linked to each other through biotin binding to an intermediate streptavidin molecule. Other intermediate linkers may be used in accordance with the present disclosure. In some embodiments, such as those where the molecule is a nucleic acid, an intermediate linker may not be required. For example, the docking strand of a BP-NA conjugate may be an extension (e.g., 5' or 3' extension) of a nucleic acid molecule such as, for example, a nucleic acid aptamer.

Pluralities of BP-NA conjugates (e.g., protein-nucleic acid conjugates) and imager strands are provided herein. A plurality may be a population of the same species or distinct species. A plurality of BP-NA conjugates of the same species may comprise conjugates that all bind to the same target (e.g., biomolecule) (e.g., the same epitope or region/domain).

Conversely, a plurality of BP-NA conjugates of distinct species may comprise conjugates, or subsets of conjugates, each conjugate or subset of conjugates binding to a distinct epitope on the same target or to a distinct target. A plurality of imager strands of the same species may comprise imager strands with the same nucleotide sequence and the same fluorescent label (e.g., Cy2, Cy3 or Cy4 or other). Conversely, a plurality of imager strands of distinct species may comprise imager strands with distinct nucleotide sequences (e.g., DNA sequences) and distinct fluorescent labels (e.g., Cy2, Cy3 or Cy4 or other) or with distinct nucleotide sequences and the same fluorescent (e.g., all Cy2 or other). The number of distinct species in a given plurality of BP-NA conjugates is limited by the number of binding partners (e.g., antibodies) and the number of docking strands of different nucleotide sequence (and thus complementary imager strands). In some embodiments, a plurality of fluorescently-labeled imager strands may contain 1 to about 200 or more distinct species of BP-NA conjugates and/or imager strands. For example, a plurality may contain at least 1, 2, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200 or more distinct species.

The present disclosure also contemplates docking strands that can bind directly to a target. For example, a docking strand may contain, in addition to imager-binding domain(s) (e.g., one, two, three, or more, with the same or distinct fluorophores), a target domain that is complementary to and binds to a target, such as, for example, mRNA or other nucleic acid.

Methods provided herein are based, in part, on the programmability of nucleic acid docking strands and imager strands. That is, for example, docking strands and imager strands can be designed such that they bind to each other under certain conditions for a certain period of time. This programmability permits transient binding of imager strands to docking strands, as provided herein. Generally, the methods provided herein are directed to identifying one or more target(s) (e.g., biomolecule(s) such as a protein or nucleic acid) in a particular sample (e.g., biological sample). In some instances, whether or not one or more target(s) is/are present in a sample is unknown. Thus, methods of the present disclosure may be used to determine the presence or absence of one or more target(s) in a sample suspected of containing the target(s). In any one of the aspects and embodiments provided herein, a sample may contain or may be suspected of containing one or more target(s).

Methods provided herein can also be used to identify the absolute quantity of a single target (e.g., such as, for example, a particular protein), or the quantity of a single target relative to one or more other targets.

Further, methods provided herein may be used to identify the location of a target within a sample or relative to other targets in the sample.

Methods provided herein may comprise, in some embodiments, contacting a sample with (a) at least one BP-NA conjugate (e.g., protein-nucleic acid conjugate) that comprises a binding partner linked to a docking strand and (b) at least one labeled, optionally fluorescently labeled, imager strand that is complementary to and transiently binds to the docking strand of the at least one BP-NA conjugate, and then determining whether the at least one BP-NA conjugate binds to at least one target (such as a biomolecule target) in the sample.

In some embodiments, the determining step comprises imaging (e.g., with time-lapsed fluorescent microscopy techniques) transient binding of the at least one labeled, optionally fluorescently labeled, imager strand to the docking strand of the at least one BP-NA conjugate.

Other methods provided herein may comprise, in some embodiments, contacting a sample with (a) at least two BP-NA conjugates, each comprising a binding partner linked to a docking strand, and (b) at least two labeled, optionally spectrally distinct, fluorescently labeled, imager strands that are complementary to and transiently bind to respective docking strands of the at least two different BP-NA conjugates, and then determining whether the at least two BP-NA conjugates bind to at least one, or at least two, targets (such as biomolecule targets) in the sample. Binding of the BP-NA conjugates to respective targets can be determined by imaging transient binding of one of the at least two labeled, optionally spectrally distinct, fluorescently labeled, imager strands to a docking strand of one of the at least two BP-NA conjugates to produce a first image, and then imaging transient binding of another of the at least two labeled, optionally spectrally distinct, fluorescently labeled, imager strands to a docking strand of another of the at least two BP-NA conjugates to produce a second image. In some embodiments, the methods further comprise combining the first image and the second image to produce a composite image of signals (e.g., fluorescent signals), wherein the signals (e.g., fluorescent signals) of the composite image are representative of the at least two targets. As used herein, a "composite image" refers to a single image produced by combining (e.g., overlaying) multiple images of the same (or substantially similar) area. A composite image may also be referred to as a super-resolution image, as described elsewhere herein.

In some embodiments, the first determining step comprises imaging transient binding of the first fluorescently-labeled imager strand to the docking strand of the first BP-NA conjugate to produce a first image, and the second determining step comprises imaging transient binding of the at least one other fluorescently-labeled imager strand to the docking strand of the at least one other BP-NA conjugate to produce a second image. In some embodiments, the methods further comprise assigning a pseudo-color to the fluorescent signal in the first image, and assigning at least one other pseudo-color to the fluorescent signal in the second image. Further still, in some embodiments, the methods comprise combining the first image and the second image to produce a composite image of the pseudo-colored signals, wherein the pseudo-colored signals of the composite image are representative of the at least two targets (e.g., biomolecule targets). All imager strands may be labeled with the same fluorophore - that is, the imager strands are spectrally indistinct. In some embodiments, the docking strands are linked to binding partners (e.g., proteins such as antibodies, or nucleic acids such as DNA or nucleic acid aptamers).

An advantage of the methods of the present disclosure is that partitioning and sequential imaging can be used to obtain multiplexed super-resolved images of up to hundreds of different species using only a single optimized fluorescent dye. Using these methods, the number of distinct nucleotide sequences (e.g., DNA sequences), as opposed to the number of spectrally distinct dyes, limits the multiplexing capability. In some methods of the present disclosure, for example, those that use an imager strand with a length of 9 nucleotides, there are several hundred species within tight bounds for binding kinetics that may be used for a single sample, representing a tremendous increase in multiplexing compared to direct "traditional" imaging approaches.

### FIGURES:

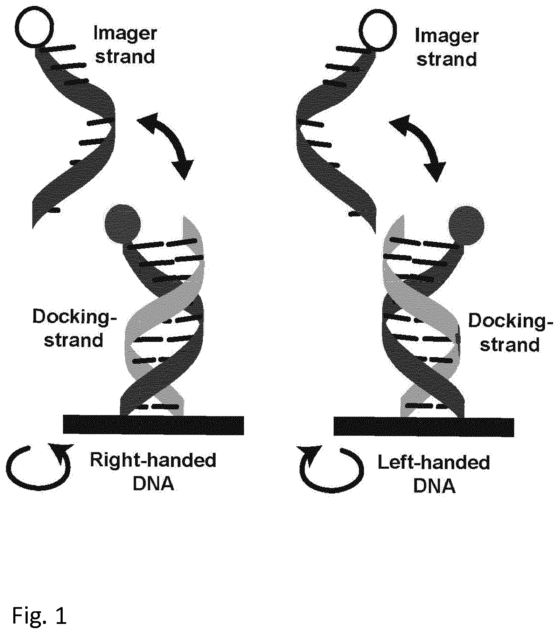

- Fig. 1: **Basic principle.** A docking strand of DNA is brought to the target structures and an imager strand is added to the solution. The imager strand transiently binds to the docking strand and only during this time is its fluorophore excited by excitation light and thus detectable for a period sufficient to localize it with an accuracy of few nanometers. This is possible with right-handed and left-handed DNA.

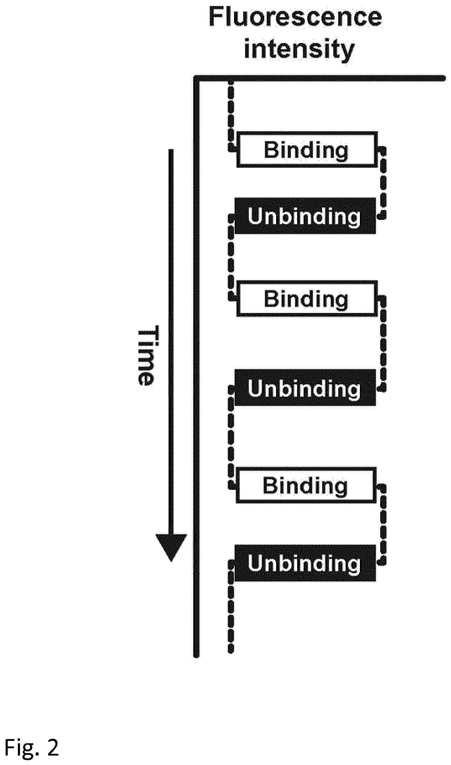

- Fig. 2: **Binding kinetics.** Because the number and nature of base pairing nucleotides determines the length of individual binding events, the length of binding events can be tuned such that many individual events can be sampled in a reasonable timeframe. Furthermore, individual binding sites can be sampled multiple times.

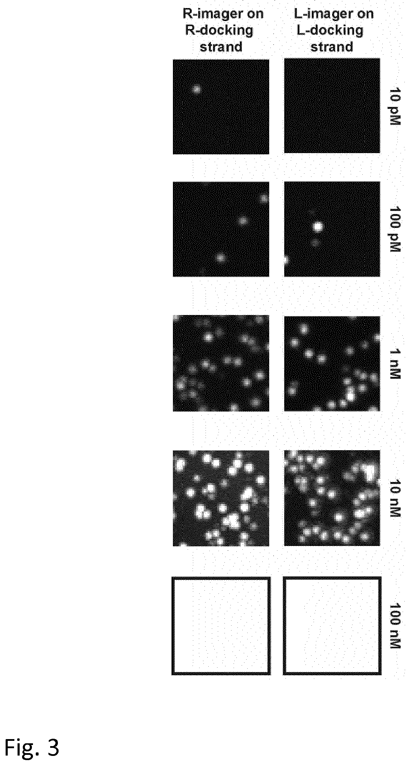

- Fig. 3: **Binding is equivalent for right-handed and left-handed DNA.** Shown are images from experiments in which docking strands have been immobilized on glass coverslips for microscopy and then increasing concentrations of fluorescence-labeled binding strands have been added. No detectable difference between R-DNA and L-DNA is observed.

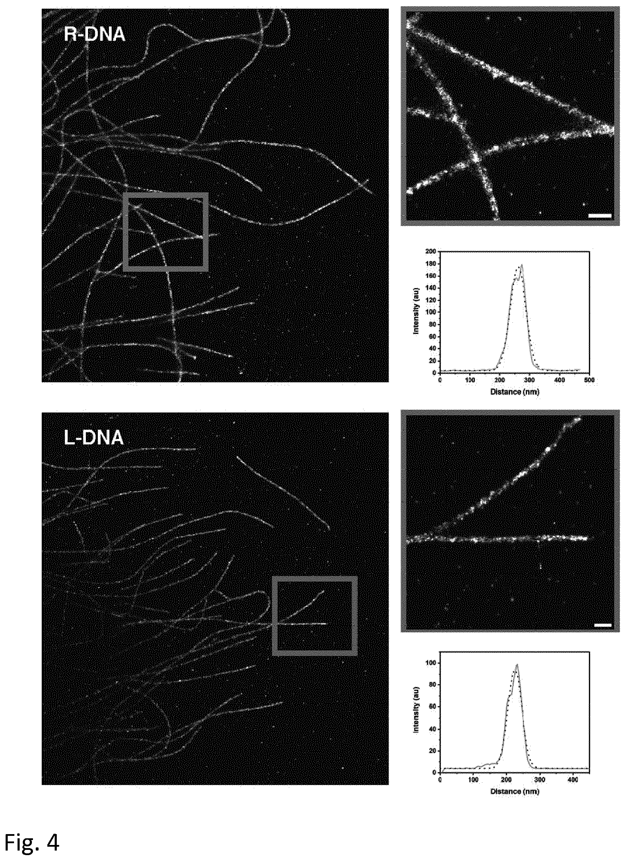

- Fig. 4: **Image rendering.** In these rendered superresolution images, an R-DNA (top) or L-DNA (bottom) docking strand has been delivered to cellular microtubules via an antibody against tubulin and the corresponding imager strand has been added to generate a superresolution image. 20'000 images have been acquired on the microscope in which individual imager strands were detected the individual detection events were fitted to yield the exact position and finally an image was rendered from all collected localizations. Again, this is possible with right-handed and left-handed DNA at equal final resolution

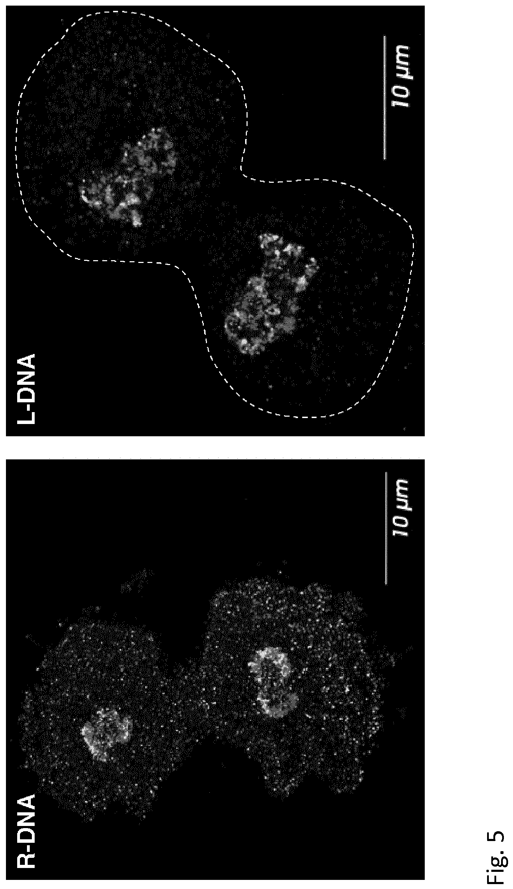

- Fig. 5: **Superiority of L-DNA PAINT over R-DNA-PAINT in imaging of nuclear structures.** Shown are two dividing cells in which the marker protein Ki67 is labeled with an R-DNA (left) or L-DNA (right) docking strand via an antibody. A superresolution image was then generated by adding an R-DNA (left) or L-DNA (right) imager strand. The background in the cell surrounding the specific Ki67 staining (two bright structures in both images) is significantly stronger for R-DNA.

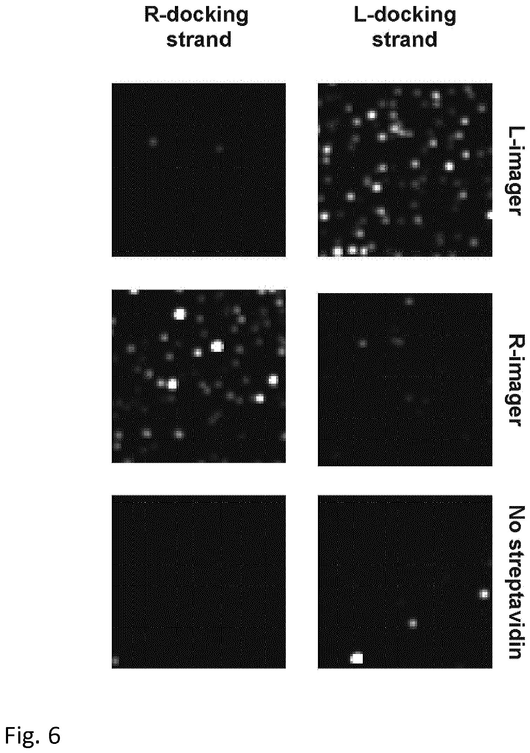

- Fig. 6: **Lack of interaction between inverse complementary R-DNA and L-DNA.** Shown are fluorescence images in which an L-DNA (top) or R-DNA (bottom) docking strand have been immobilized on microscopy coverglass via biotin-streptavidin linkage. Afterwards, dye-coupled imager strands were added and samples were imaged. The docking-strands and imager-strands were chosen such that the nucleotide sequence of the docker strands and the imager strands were inverse complementary to one another and could thus bind to one another. On the other hand, the sequence of the docking strands was identical for L- and R-DNA and the sequences of the imager strands were identical for L-and R-DNA. When L-imager strands were added to L-docking strands, binding was detected, but not when they were added to R-docking strands of the same sequence (top row). Likewise, when R-imager strands were added to R-docking strands, binding was detected, but not when they were added to L-docking strands of the same sequence. When nothing was attached to the coverglass, neither imager strand bound to the coverglass. Binding of L-DNA is thus specific for L-DNA and of (naturally occurring) R-DNA to R-DNA.

### EXAMPLE Application:

### L-DNA mediated superresolution imaging of antigen located to the cell nucleus.

DNA-PAINT is a method by which single molecules are sequentially imaged via the transient binding of a DNA-oligomer coupled to a fluorescent dye to its reverse complimentary counterpart. This is theoretically possible for left handed as well as right-handed DNA (Figures 1, 2). To test, whether L-DNA is indeed equally suited for DNA-PAINT as R-DNA, we cleaned Labtek chamber (Ibidi) by 15 minutes of sonication in 1 M KOH followed by 10 washing steps in ultrapure water and air drying. We then added a 5:1 mixture of bovine serum albumin (BSA) and biotinylated BSA in 10 mM Tris-HCI buffer, pH 7.5 containing 100 mM NaCI and 0.05 % Tween-20 (surface buffer) for 3h at room temperature to generate a biotinylated surface. This surface was then modified by incubation for 20 minutes with 0.2 mg/ml of newly purchased neutravidin (Sigma) at room temperature in surface buffer. After three subsequent washes with PBS, we added a 500 pM solution of biotinylated binder oligomer of either L-DNA of R-DNA (sequence: TTTCTTCATTA) for 30 minutes in binding buffer (5 mM Tris-HCI, pH 8.0, 10 mM MgCl₂, 1mM EDTA and 0.05 % Tween-20). After three washes in PBS, the samples containing surface-bound L-DNA or R-DNA were imaged on a Vutara 532 microscope (Bruker) after the respective addition of Atto647N-coupled L-DNA or R-DNA oligomers of a reverse complement sequence (GTAATGAAGA, reverse complement sequence underlined) in concentrations ranging from 10 pM to 100 nM in PBS pH 8.0 supplemented with 500 mM NaCl. We found that the L-DNA sequence coupled to a dye exhibits the exact same binding characteristics to its reverse complement counterpart as R-DNA with the same sequence of bases does, showing that non-natural L-DNA and natural R-DNA are functionally equivalent for imaging (Figure 3).

To test, whether L-DNA was suitable for DNA-PAINT in cells as well, we plated HeLa cell (ATCC) on 18 mm round coverslips in Dulbecco's modification of Eagle's Medium (DMEM( containing 10% fetal calf serum and 1 % Glutamax (all ThermoFisher) at 37 °C in a humidified incubator. We washed the cells 3 times with PEM buffer (0.1M PIPES, pH 6.8, 2 mM EGTA, 1mM MgSO₄) at 37 °C and permeabilized them for 30 seconds in BRB80 (80 mM PIPES, pH 6.8, 10 mM EGTA, 1mM MgCl₂) contining 0.2 % Triton-X-100. Afterwards, the cells were fixed in 3.2 % paraformaldehyde and 0.1 % glutaraldehyde in BRB80 for 10 min at 37 °C and washed with PEM buffer. We then quenched with 1 mg/ml NaBH₄ in PBS for 7 min at room temperature and washed the fixed cells 3 times with PEM buffer. The samples were blocked in PEM containing 4% goat serum, 1% BSA for 1 hour and incubated with 2 drops of ImagelT (ThermoFisher) for 30 minutes at room temperature. Afterwards, the sample was washed 3 times with PEM buffer and stained with monoclonal anti-alpha-tubulin antibody produced in mouse (Sigma T5618) in a 1:1000 dilution in PEM buffer supplemented with 1% BSA and 0.1 % TX-100 over night at 4 °C. Subsequently, the sample was washed 3 times with PEM buffer and stained with a biotinylated anti-mouse secondary antibody produced in goat (ThermoFisher) in 5% BSA and 0.1 % Triton X-100 in PBS and washed 3 times in PEM buffer again. Then, 0.2 mg/mL freshly prepared streptavidin (Sigma) was added in PBS for 30 minutes at room temperature and washed 3x with PBS. Subsequently, 500 pM of the same biotinylated left- or right-handed binder DNA oligo's as used for the in vitro binding assay were added for 30 min in PBS at room temperature. Then the sample was washed a final 3 times in PBS and imaged with 500 pM left- or right-handed imager DNA oligo's coupled Atto647N (as used for the in vitro binding assay). Imaging was performed on a Vutara 532 microscope (Bruker) equipped with a Hamamatsu ORCA Flash4.0 sCMOS for super-resolution imaging and a 60x oil immersion TIRF objective with numerical aperture 1.49 (Olympus). Data was required with TIRF/HILO-illumination at a laser power density of -2.5 kW/cm² using a 639 nm laser line. Images were typically collected at 300 ms acquisition time and typically 20000 images were collected to reconstruct super-resolution composite images (Figure 4). In both conditions, high quality superresolution data could be acquired. We concluded that L-DNA performs at least equally well to R-DNA in DNA-PAINT.

To test, whether DNA-PAINT using L-DNA would indeed lead to less background resulting from endogenous DNA that DNA-PAINT using R-DNA, we performed immunofluorescence staining like against tubulin described above, but against a nuclear antigen with either L-DNA or R-DNA (Figure 5). We found that the L-DNA based procedure led to substantially less background than the R-DNA-based procedure. We concluded that non-natural nucleotide based DNA-PAINT is prone to significantly less background than R-DNA based DNA-PAINT.

Finally, we attached our biotinylated L-DNA and R-DNA docking strands to cleaned coverslips via biotinylated BSA and neutravidin like described above for Figure 3. When we then added R-DNA imager strands coupled to Atto647N at 10 nM in PBS/500 mM NaCI and imaged the sample on the Vutara532 microscope, they were detectable on the R-DNA docking strands, but not on the L-DNA docking strands or on empty controls (Figure 6). Likewise, L-DNA imager strands coupled to Atto647N added at 10 nM in PBS/500 mM NaCI could be detected on L-DNA docking strands, but not on R-DNA docking strands or empty controls (Figure 6). We concluded that L-DNA cannot bind to reverse complementary R-DNA, but to reverse complementary L-DNA only.

## Claims

1. Method for optical imaging by delivery of a dye via a base-pairing mechanism, wherein a first strand being delivered to a target structure, and a complimentary second strand carrying a dye molecule **characterized in that** the two pairing strands comprise non-naturally occurring oligonucleotide.

2. Method according to claim 1, **characterized in that** the non-naturally occurring oligonucleotide is a left handed oligonucleotide, preferably a left handed DNA.

3. Method according to one of the preceding claims, **characterized in that** the non-naturally occurring oligonucleotide is built by replacing units or ions of the backbone by derivatives thereof or chemical compatible ions, especially replacing phosphor-ions by arsenic-ions.

4. Method according to one of the preceding claims, **characterized in that** the first strand is an adapter strand which is capable of transiently binding to the second strand.

5. Method according to one of the preceding claims, **characterized in that** the second strand is an imager strand carrying a dye, wherein the label comprises one of a fluorophene, a phosphorescene, a quantum dot, a nanoparticle, a nanodiamond or other optically detectable label.

6. Method according to one of the preceding claims, **characterized in that** the dye is imaging active if the second strand is linked to a corresponding first strand, and optionally imaging inactive if the second strand is not linked to a corresponding first strand.

7. Method according to one of the preceding claims, **characterized in that** the complementary labeled imager strand is about 4 to about 30 nucleotides, especially 7 to 20, preferably 8 to 10 in length.

8. Method according to one of the preceding claims, **characterized in that** a further pair of imager and adapter strands are present in the method which differs from the first pair in the target structure and the nucleotide sequence.

9. Method according to one of the preceding claims, **characterized in that** the target is linked to the first strand through an intermediate linker.

10. Method according to one of the preceding claims, **characterized in that** the intermediate linker comprises biotin and/or streptavidin.

11. Method according to one of claims 4 to 10, wherein the adapter strand is bound to a target-recognizing antibody or nanobody.

12. Method according to one of the preceding claims, **characterized in that** the target-recognizing antibody or nanobody provides a biotin binding site, a streptavidin binding to biotin-bound antibody or nanobody , whereby the adapter strand with a biotin binding site is capable to strongly bind to the streptavidin.

13. Method according to one of the preceding claims, **characterized in that** the strand's linkage between the pair of first and second strand initiates a hybridization of the dye with the resulting DNA double strand, which initiates a detectable signal, specifically a fluorescent light having a specific wavelength.

14. A protein-oligonucleotide conjugate comprising a target linked to an adapter strand that is capable of transiently binding to a complementary labeled imager strand, **characterized in that** the adapter strand and the imager strand comprise non-naturally occurring oligonucleotide.

15. A target bound to at least one protein-oligonucleotide conjugate according to claim 14.

16. Kit comprising a first and a second strand as defined in a method of one of claims 1 to 15 and a manual for conducting a method of one of claims 1 to 15.
